# EUROPÄISCHE PATENTSCHRIFT

(11) **EP 2 440 142 B1**
(45) Veröffentlichungstag und Bekanntmachung des Hinweises auf die Patenterteilung: **23.05.2018**
(21) Anmeldenummer: 10721626.9
(22) Anmeldetag: 03.06.2010
(51) Int. Cl.: A61B 17/00

(54) **OKKLUDER**
OCCLUDER
DISPOSITIF D'OCCLUSION

(30) Priorität: 10.06.2009 CH 899092009
(43) Veröffentlichungstag der Anmeldung: 18.04.2012
(73) Patentinhaber: Carag AG, 6340 Baar (CH)
(72) Erfinder: STEINER, Claudio, 6430 Schwyz (CH); WEISHAUPT, Andreas, 6340 Baar (CH); HUMMEN, Jörg, 8802 Kilchberg (CH)
(74) Vertreter: Clerc, Natalia
(86) Internationale Anmeldenummer: PCT/CH2010/000147
(87) Internationale Veröffentlichungsnummer: WO 2010/142051

(56) Entgegenhaltungen:
- EP-A- 1 149 561
- EP-A- 1 994 887
- WO-A-01/21246
- WO-A-97/16119
- DE-U1- 29 500 381
- US-A1- 2005 267 524
- US-A1- 2007 129 755
- US-B1- 6 712 836

## Beschreibung

### TECHNISCHES GEBIET

Die vorliegende Erfindung betrifft einen Okkluder gemäss Oberbegriff des Patentanspruchs 1.

### STAND DER TECHNIK

Okkluder sind Implantate, die zum Verschliessen von Durchgängen in Kreislaufsystemen, wie beispielsweise Blutgefässen oder Shuntverbindungen eingesetzt werden und üblicherweise über eine in eine Vene eingeführte Schleuse positioniert und expandiert werden. Okkluder dienen beispielsweise zum Verschluss eines persistierenden Ductus arteriosus (PDA), eines Vorhofseptumdefekts (ASD) oder eines Ventrikelseptumdefekts (VSD). Andere Anwendungen im menschlichen und tierischen Körper sind möglich. Ihre Form ist jeweils der entsprechenden Anwendung angepasst.

Im Stand der Technik sind die unterschiedlichsten Ausgestaltungsformen von Okkludern bekannt. Sie können beispielsweise als Spiralfeder ausgebildet sein oder sich wie ein Regenschirm aufspannen lassen. Sie können ferner den Durchgang nur von einer Seite oder von beiden Seiten verschliessen. Diese Okkluder lassen sich üblicherweise in eine langgestreckte Form bringen, damit sie mittels des Katheters zum Durchgang, welcher verschlossen werden soll, gebracht werden können. Dort wird der Okkluder freigesetzt und er nimmt entweder selbsttätig oder geführt seine expandierte Gebrauchsform ein.

Ein Okkluder, welcher sich geführt öffnen lässt und somit optimal im Durchgang platziert werden kann, ist in WO 2005/074813 offenbart.

US 2005/0251154 offenbart einen Okkluder mit zwei Spiralfedern, welche auf je einer Seite des Durchgangs zu liegen kommen und den Okkluder in dieser Lage fixieren. Die Spiralfedern sind an je einem Ende über einen Bügel miteinander verbunden.

US 6 117 159 beschreibt einen Okkluder in Form eines Hohlzylinders. Der Hohlzylinder ist in Längsrichtung geschlitzt ausgebildet, wobei die durchgehenden Schlitze beabstandet zu den zwei Enden des Zylinders enden und auch den mittleren Bereich frei lassen. Dadurch entstehen flächige Streben. An den Enden der Streben und in ihrem Mittelbereich sind über dem Umfang verlaufende Kerben vorhanden, welche als Filmscharniere wirken. Werden nun die zwei Enden des Hohlzylinders einander angenähert, so klappen die Streben nach aussen und führen den Okkluder in eine expandierte Erscheinungsform über mit zwei einander gegenüberliegenden Fixierungsebenen. Ähnliche Anordnungen sind in WO 2009/045705 und WO 2005/112779 dargestellt.

Auch EP 1 836 969 zeigt einen Okkluder, bei welchem die Fixierungsstreben mit Scharnieren versehen sind.

In EP 0 474 887 weist der Okkluder zwei einander gegenüberliegende textile Kreisflächen mit einem Rahmen auf. Die zwei Kreisflächen sind über Fäden miteinander verbunden.

WO 2005/034723 offenbart ebenfalls einen Okkluder mit zwei Membranen, welche beabstandet zueinander auf einem zentralen Zylinder angeordnet sind. Drähte erstrecken sich vom Rahmen der ersten Membran zum Rahmen der zweiten Membran, wobei sie den zentralen Zylinder durchsetzen.

EP 1 149 561 und WO 97 / 46 119 beschreiben je einen Okkluder mit zwei einander gegenüberliegenden Flächen, die aus einem Rahmen und einer Membrane bestehen. Die Flächen sind miteinander verbunden. Defekte in der Kammerscheidewand oder Kammertrennwand (Ventrikelseptum) unterscheiden sich wesentlich von Defekten im Vorhof. In der Kammerscheidewand ist ein Okkluder einem grösseren Druck ausgesetzt. Des Weiteren variiert die Wandstärke mehr als im Vorhof. Sie ist insbesondere teilweise beträchtlich grösser als im Vorhof. Sie kann beim erwachsenen Menschen üblicherweise bis zu 15 mm betragen. Zudem sind andere und deutlich beengtere Platzverhältnisse als im Vorhof zu berücksichtigen. Die im Stand der Technik bekannten Okkluder sind diesen Gegebenheiten oft nicht optimal angepasst.

EP 1 149 561 offenbart einen Okkluder für septale Defekte. Der Okkluder weist zwei Membranen auf, welche jeweils mit einem Rahmen verbunden sind. Die zwei Rahmen sind über Ösen, welchen zusammengenäht sind, miteinander verbunden.

WO 97/16119 zeigt einen Okkluder mit zwei Schirmen, welche durch Drähte, die gemeinsam in ihrer Mitte zusammengehalten sind, aufgespannt werden.

US 2005/0267524 beschreibt einen Okkluder mit einer röhrenähnlichen eintückigen Stützstruktur, deren Enden geschlitzt ausgebildet sind und dadurch Arme bilden. Diese Arme können durch elastische Bänder miteinander verbunden sein.

### DARSTELLUNG DER ERFINDUNG

Es ist eine Aufgabe der Erfindung, einen Okkluder zu schaffen, welcher insbesondere zum Verschluss eines Ventrikelseptumdefekts (VSD) geeignet ist.

Diese Aufgabe löst ein Okkluder mit den Merkmalen des Patentanspruchs 1.

Der erfindungsgemässe Okkluder zum Verschliessen eines Durchgangs in einem Kreislaufsystem weist eine expandierbare Fixierungseinheit zur Fixierung des Okkluders beim Durchgang auf, wobei der Okkluder im Durchgang von einer kompakten Erscheinungsform in eine expandierte Erscheinungsform überführbar ist. Der Okkluder weist ein distales und ein proximales Axialteil auf, in welchen die Fixierungseinheit schwenkbar gehalten ist. Erfindungsgemäss weist die Fixierungseinheit distale Fixierungsarme und proximale Fixierungsarme auf, wobei die distalen Fixierungsarme im distalen Axialteil und die proximalen Fixierungsarme im proximalen Axialteil schwenkbar gehalten sind. Die distalen und die proximalen Fixierungsarme weisen freie Enden auf, welche über relativ zu den Fixierungsarmen bewegbare Verbindungsglieder miteinander verbunden sind, wobei je ein distaler Fixierungsarm mit je einem proximalen Fixierungsarm verbunden ist. Vorzugsweise sind jeweils diametral einander gegenüberliegende Fixierungsarme miteinander verbunden.

Proximal bedeutet hier dem Arzt zugewandt und distal dem Arzt abgewandt, also patientenseitig.

Dieser Okkluder hat den Vorteil, dass er sowohl im kompakten, langgezogenen Zustand wie auch im expandierten Zustand relativ geringe Abmessungen aufweist und trotzdem dem hohen Druck im Bereich der Kammerscheidewand standhalten kann. Ein weiterer Vorteil ist, dass der Okkluder in expandierter Gebrauchslage relativ flach beidseitig an der Kammerscheidewand anliegt, nicht oder kaum in die Kammer hineinragt und die Herzklappenfunktion nicht stört. Vorteilhaft ist ferner, dass sich der Okkluder geführt öffnen bzw. expandieren und somit optimal im Durchgang platzieren lässt.

Im kompakten Zustand ist der erfindungsgemässe Okkluder im Vergleich zu den bekannten Okkludern relativ kurz ausgebildet. Typische Längen liegen bei 20 mm bis 50 mm, vorzugsweise 30 mm. Dadurch lässt sich der Okkluder bei der Einführung in die Kammerscheidewand besser um enge Bogen führen.

Im expandierten Zustand beträgt sein Durchmesser circa 10 mm bis 30 mm, vorzugsweise 20 mm. Auch dieser Durchmesser ist somit üblicherweise kleiner als die Durchmesser der bekannten, für denselben Zweck eingesetzten Okkluder.

In einer bevorzugten Ausführungsform verlaufen die Verbindungsglieder zwischen den einzelnen distalen und proximalen Fixierungsarmen unabhängig voneinander. Sie kreuzen sich vorzugsweise in einem Bereich. Dieser Bereich liegt in Längsrichtung des Okkluders vorzugsweise annähernd in der Mitte zwischen dem proximalen Axialteil und dem distalen Axialteil. In einer ersten Ausführungsform ist dieser Kreuzungsbereich frei von weiteren Elementen. In einer anderen Ausführungsform verlaufen die Verbindungsglieder in diesem Bereich einzeln verschiebbar in einer sie gemeinsam umgebenden Hülse.

In einer bevorzugten Ausführungsform ist jeder der Fixierungsarme bogen- oder u-förmig ausgebildet mit zwei Schenkeln und mit einem diese Schenkel verbindenden Steg. Die zwei Schenkel weisen freie Enden auf, welche im proximalen oder distalen Axialteil schwenkbar gehalten sind. Der Steg bildet dabei das besagte freie Ende des Fixierungsarms. Diese u-förmige Ausgestaltung gibt den Fixierungsarmen Stabilität, sie lassen sich einfach montieren und einfach mit den Verbindungsgliedern verbinden.

Vorzugsweise ist der Steg von einer Hülse umfasst, wobei der Steg schwenkbar in der Hülse gehalten ist. An jeder Hülse ist ein Verbindungsglied befestigt, vorzugsweise formschlüssig verbunden oder verschweisst. Dadurch ist auf einfache Weise ein Gelenk oder ein Scharnier geschaffen, welches die Relativbewegung zwischen Fixierungsarm und Verbindungsglied ermöglicht. Vorzugsweise ist der Steg annähernd geradlinig ausgeführt, so dass die Hülse ohne weitere Hilfsmittel in ihrer Lage auf dem Fixierungsarm gehalten ist. Ist der Steg rund ausgebildet, lässt sich die Hülse beispielsweise durch zwei Klemmhülsen in ihrer Lage fixieren. Diese zwei Klemmhülsen sind je auf einer Stirnseite der Scharnierhülse angeordnet.

In einer bevorzugten Ausführungsform bestehen die Fixierungsarme aus Drähten, vorzugsweise aus Nitinol oder einem resorbierbaren Material. Die Verbindungsglieder können ebenfalls aus Drähten oder aus Schnüren bestehen.

Bestehen die Verbindungsglieder aus Drähten, so unterstützen sie die Bewegung der Fixierungsarme während der Expansion und zwingen die Fixierungsarme in ihre geöffnete Stellung.

Bestehen die Verbindungsdrähte aus Schnüren, beispielsweise aus Polypropylen (PP) oder Polylactat, so begrenzen sie den maximalen Abstand zwischen den distalen und proximalen Fixierungsarmen und begrenzen auch den Expansionswinkel der Fixierungsarme. Die Fixierungsarme öffnen sich in dieser Ausführungsform ohne weitere Hilfe. Sie sind hierfür unter einem Winkel im distalen und im proximalen Axialteil gelagert. Dieser Winkel ist im kompakten, durch äussere Krafteinwirkung erzielten Zustand verkleinert. Bei Fehlen dieser äusseren Krafteinwirkung wird der Winkel durch die Federwirkung bzw. Elastizität der Drähte wieder eingenommen.

Das proximale Axialteil und das distale Axialteil sind vorzugsweise als miteinander verbindbare Kopplungsteile ausgebildet, welche ineinander steckbar und fixierbar sind. Dadurch fixieren sie den Okkluder in seiner expandierten Erscheinungsform wobei der Abstand zwischen den distalen und proximalen Fixierungsarmen vom Arzt gewählt werden kann.

Vorzugsweise sind die distalen und proximalen Fixierungsarme gleich gross ausgebildet und der Okkluder ist bezüglich der Fixierungseinheit spiegelsymmetrisch ausgebildet. Der Okkluder kann jedoch auch asymmetrisch ausgebildet sein oder die Fixierungsarme der distalen und/oder proximalen Seite können untereinander unterschiedliche Grössen und/oder Formen aufweisen.

Des Weiteren können anstelle der Drähte der Fixierungsarme auch flächige Bügel oder Arme eingesetzt werden. Die Drähte können auch andere Formen anstelle der U-Form aufweisen.

Vorzugsweise weist der Okkluder mindestens einen Verschlusskörper zum Verschliessen des Durchgangs auf. Dieser Verschlusskörper ist vorzugsweise eine Membran, ein Tampon oder ein Ballon. Ist er eine Membran, so sie vorzugsweise mittels der Fixierungsarme in ihre expandierte Erscheinungsform bringbar. Der Tampon ist vorzugsweise komprimierbar und elastisch ausgebildet, so dass er selbsttätig seine expandierte Form annehmen kann. Ist der Verschlusskörper ein Ballon, so erstreckt sich dieser vorzugsweise vom proximalen Axialteil bis zum distalen Axialteil und ist an diesen zwei Teilen befestigt. Er umgibt die Fixierungsarme und die Verbindungsglieder und wird vorzugsweise von den Fixierungsarmen in seine expandierte Stellung gebracht.

Vorzugsweise ist der Verschlusskörper, insbesondere der Tampon, in einem Bereich angeordnet, welcher von einer Seite von den distalen Fixierungsarmen und an einer gegenüberliegenden Seite von den proximalen Fixierungsarmen definiert ist. Dadurch befindet sich der Verschlusskörper im Durchgang selber und nicht an einer Aussenseite des Durchgangs. Diese Anordnung des Verschlusskörpers lässt sich auch in anderen als den hier beschriebenen Okkludern einsetzen und wird hier als eine davon unabhängige Erfindung beansprucht. Ein Okkluder mit mindestens einem derart angeordneten Verschlusskörper erlaubt einen schnellen und optimalen Verschluss auch bei hohen Drücken. Vorteilhaft ist, dass er nicht oder kaum in die Kammer hineinragt.

Die Verbindungsglieder definieren den maximalen Abstand zwischen den proximalen und distalen Fixierungsarmen und unterstützen die Expansion des Okkluders. In einer Ausführungsform sind jedoch keine Verbindungsglieder vorhanden. Diese Ausführungsform wird ebenfalls als unabhängige Erfindung beansprucht, wobei sie alle oben genannten Varianten und Kombinationen ohne Verbindungsglieder aufweisen kann.

Diese erfindungsgemässen Okkluder eignen sich insbesondere zum Verschluss eines Ventrikelseptumdefekts (VSD). Sie lassen sich jedoch auch in anderen Bereichen, insbesondere den oben genannten Bereichen, einsetzen. Sie ermöglichen einen anatomisch und physiologisch optimierten Verschluss eines Defekts.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung eines menschlichen Herzens mit einem Defekt in der Kammerscheidewand (VSD);
- Figur 2: eine perspektivische Darstellung einer ersten Ausführungsform des erfindungsgemässen Okkluders ohne Verschlusskörper, in teilweise expandiertem Zustand;
- Figur 3: eine Seitenansicht des Okkluders gemäss Figur 2 mit Verschlusskörper, in annähernd langgezogenem Zustand;
- Figur 4: eine Seitenansicht des Okkluders gemäss Figur 2 mit Verschlusskörper, in teilweise expandiertem Zustand;
- Figur 5: ein Querschnitt in vergrösserter Darstellung durch das distale Ende des Okkluders gemäss Figur 2 in einer Seitenansicht;
- Figur 6: eine Seitenansicht des Okkluders gemäss Figur 2 mit einem teilweise dargestellten Kathetersystem;
- Figur 7: eine Seitenansicht einer zweiten Ausführungsform des erfindungsgemässen Okkluders im annähernd langgezogenen Zustand mit einer Ballonhülle;
- Figur 8: den Okkluder gemäss Figur 7 in teilweise expandiertem Zustand;
- Figur 9: eine Seitenansicht einer dritten Ausführungsform des erfindungsgemässen Okkluders im annähernd langgezogenen Zustand mit Membranen;
- Figur 10: den Okkluder gemäss Figur 9 in teilweise expandiertem Zustand;
- Figur 11: eine Seitenansicht einer vierten Ausführungsform des erfindungsgemässen Okkluders in teilweise expandiertem Zustand mit Verbindungsschnüren;
- Figur 12: den Okkluder gemäss Figur 3, eingeführt in einem Durchgang und
- Figur 13: den Okkluder gemäss Figur 12 im expandierten und fixierten Zustand.

### BESCHREIBUNG BEVORZUGTER AUSFÜHRUNGSFORMEN

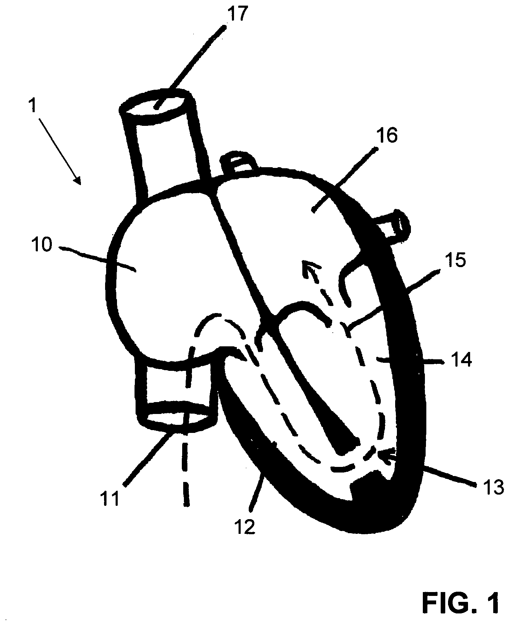

In Figur 1 ist ein menschliches Herz 1 mit einem Ventrikelseptumdefekt (VSD) dargestellt. Die rechte Vorhof ist mit der Bezugsziffer 10 versehen, die untere Hohlvene mit 11, die rechte Kammer mit 12, der Defekt in der Kammerscheidewand mit 13, die linke Kammer mit 14, der linke Vorhof mit 16 und die obere Hohlvene mit 17. Der gestrichelte Pfeil 15 bezeichnet den Weg, über welche ein Führungsdraht eingeführt wird. Dadurch lässt sich ein Okkluder mittels eines Kathetersystem an die Stelle des Defekt bringen und er kann den Defekt verschliessen.

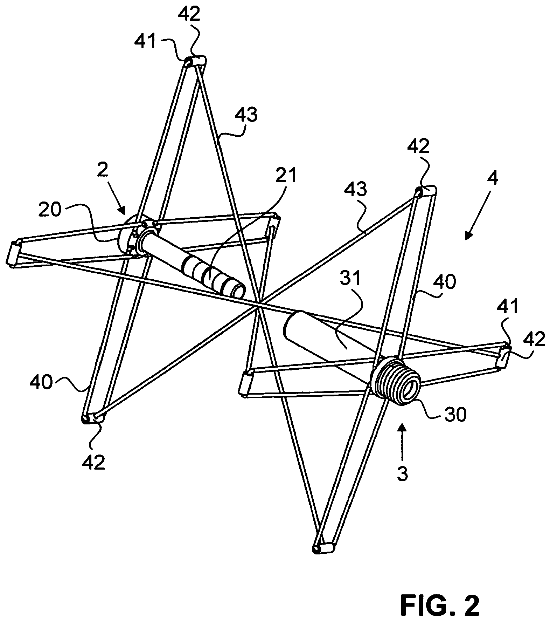

In den Figuren 2 bis 6 ist ein erstes Ausführungsbeispiel des erfindungsgemässen Okkluders dargestellt. In Figur 2 ist er in einer Stellung zwischen kompaktem Zustand und expandiertem Zustand dargestellt. Er weist eine hier nicht dargestellte virtuelle Längsachse auch. Entlang dieser Längsachse sind ein distales Axialteil 2 und ein proximales Axialteil 3 angeordnet. Die zwei Axialteile dienen in den hier beschriebenen Beispielen als Kopplungsteile, welche ineinander steckbar sind und so den Okkluder in seiner expandierten Stellung fixieren.

Das distale und das proximale Kopplungsteil 2, 3 sind vorzugsweise aus einem Kunststoff gefertigt.

Das distale Kopplungsteil 2 weist ein distales Endstück 20 auf, welches stirnseitig offen ausgebildet ist. An diesem Endstück 20 ist ein Einsteckschaft 21 angeformt, welcher einen geringeren äusseren Durchmesser aufweist als das Endstück 20. An seinem freien Ende ist er vorzugsweise mit Rückhalterippen oder -nasen versehen. Der Einsteckschaft 21 ist hohl ausgebildet.

Das proximale Kopplungsteil 3 ist durchgehend hohl ausgebildet und weist eine Eingangs- und eine Ausgangsöffnung auf. Ein proximales Endstück 30 des Kopplungsteils 3 weist ein Aussengewinde auf. An dieses grenzt eine Aufnahmehülse 31 an, welche einen grösseren Durchmesser aufweist als der Einsteckschaft 21. Der Einsteckschaft 21 ist in diese Aufnahmehülse 31 einsteckbar und dank den Rückhalterippen in seiner Lage fixierbar. Vorzugsweise sind mehrere, hier drei Einrastpositionen möglich, so dass der Abstand zwischen dem distalen und proximalen Endstück wählbar ist.

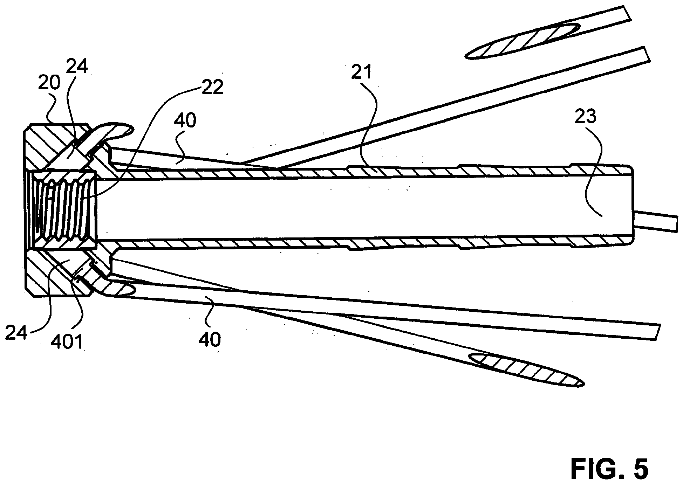

Das distale und das proximale Kopplungsteil 2, 3 weisen im Bereich zwischen Endstück 20, 30 und Schaft bzw. Hülse 21,31 Aufnahmeöffnungen 24 (siehe Figur 5) zur Aufnahme von Fixierungsarmen 40 auf.

Diese Fixierungsarme 40 sind Teil einer aufklappbaren oder expandierbaren Fixierungseinheit 4. Die Fixierungsarme 40 sind als Bügel ausgebildet und weisen zwei Schenkel und einen diese zwei Schenkel verbindenden Steg auf. Die zwei Schenkel verlaufen vorzugsweise annähernd parallel zueinander. Der Steg ist vorzugsweise geradlinig ausgebildet. Die freien Enden der Schenkel sind in den Aufnahmeöffnungen 24 des distalen und des proximalen Kopplungsteils 2, 3 schwenkbar gelagert. Der Schenkel bildet das freie Ende des Fixierungsarms.

Ein Beispiel für eine derartige Lagerung ist in Figur 5 erkennbar. Andere Lagerungen sind möglich. Es ist das distale Kopplungsteil 2 dargestellt. Das proximale Kopplungsteil 3 ist bezüglich der Lagerung der Arme 40 gleich ausgebildet. Im Kopplungsteil 2 sind über dem Umfang verteilt Aufnahmeöffnungen 24 in Form von gestuften Löchern vorhanden. Die Längsachse der Öffnungen 24 verläuft in einem Winkel zur Längsachse des Okkluders und entspricht vorzugsweise der Winkelstellung der Arme 40 in ihrer expandierten Position. Die Schenkel der Arme 40 sind in dieser Figur gebogen dargestellt. Vorzugsweise sind sie jedoch einstückig ohne Bogen ausgebildet.

Das Ende wird durch eine Erweiterung in Form eines Nagelkopfes 401 gebildet. Dieser Nagelkopf 401 wird in die Stufe der Öffnung 24 eingehängt, so dass der Arm 40 in der Öffnung bewegbar fixiert ist.

Wie in Figur 2 erkennbar ist, kreuzen sich die Schenkel der Arme 40 in ihrem Endbereich, d.h. ein Schenkel eines ersten Arms kreuzt einen Schenkel eines zweiten Arms im Bereich ihrer Befestigungsstellen. Zwischen den zwei Bügeln eines Arms 40 ist in diesem Beispiel je ein Schenkel von zwei benachbarten Armen 40 befestigt.

Vorzugsweise sind auf der distalen und auf der proximalen Seite des Okkluders je vier derartiger Fixierungsarme 40 vorhanden. Es können jedoch auch drei oder fünf oder eine andere Anzahl Arme 40 vorhanden sein. Es kann auch auf beiden Seiten eine unterschiedliche Anzahl Arme 40 vorhanden sein.

Vorzugsweise sind alle Arme 40 gleich lang und gleich breit ausgebildet. Die Arme 40 der proximalen Seite können sich jedoch von denjenigen der distalen Seite unterscheiden, beispielsweise in Grösse und / oder Form. Des Weiteren können sich die Arme derselben Seite voneinander unterscheiden.

Der Steg ist von einer Hülse 42 umgeben, welche sich relativ zum Steg um ihre Längsachse drehen kann. An dieser Hülse 41 ist ein Verbindungsglied 43 angeordnet. Es ist beispielsweise mit ihm verschweisst oder an ihm angeklebt. Vorzugsweise ist es in eine Bohrung eingesteckt und mit einem Nagelkopf in Position gehalten.

Vorzugsweise ist jeder Steg mit einer Hülse 42 und jede Hülse 42 ist mit einem, vorzugsweise mit genau einem Verbindungsglied 43 versehen.

Jedes Verbindungsglied 43 verbindet einen distalen Fixierungsarm 40 mit einem proximalen Fixierungsarm 40. Vorzugsweise erfolgt diese Verbindung zwischen zwei diametral gegenüberliegenden Fixierungsarmen 40, so dass sich die Verbindungsglieder 43 kreuzen. Sie verlaufen jedoch auch im Kreuzungsbereich unabhängig voneinander. Sie können in diesem Bereich lose über eine Hülse verbunden sein, welche jedoch ihre voneinander getrennte Längsverschiebbarkeit innerhalb der Hülse und relativ zu ihr nicht behindert. Je nachdem, ob die proximale und distale Seiten der Fixierungsarme symmetrisch ausgebildet sind oder nicht, befindet sich der Kreuzungsbereich in der Mitte zwischen dem distalen und dem proximalen Endstück 20, 30 des Okkluders oder näher beim einen oder anderen dieser Endstücke 20, 30.

Die Fixierungsarme 40 sind vorzugsweise aus Draht gefertigt. Bevorzugt ist Nitinol oder ein resorbierbares Material. Bevorzugte Durchmesser der Arme 40 betragen beispielsweise 0.1 mm bis 0.3 mm, vorzugsweise 0.2 mm. Die Länge der Arme 40, gemessen vom Bogen 40 bis zum Steg beträgt beispielsweise 10 mm bis 30 mm, vorzugsweise 20 mm.

In diesem Beispiel bestehen auch die Hülse 42 und die Verbindungsglieder 43 aus einem Draht, vorzugsweise aus Nitinol oder einem resorbierbaren Material. Vorzugsweise sind die Verbindungsdrähte 43 gleich dick ausgebildet wie die Fixierungsarme 40. Sie können beispielsweise einen Durchmesser von 0.1 mm bis 0.3 mm, vorzugsweise von 0.2 mm aufweisen. Ihr Länge beträgt beispielsweise 5 mm bis 15 mm, vorzugsweise 10 mm.

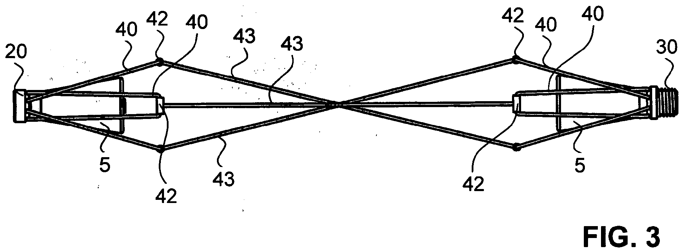

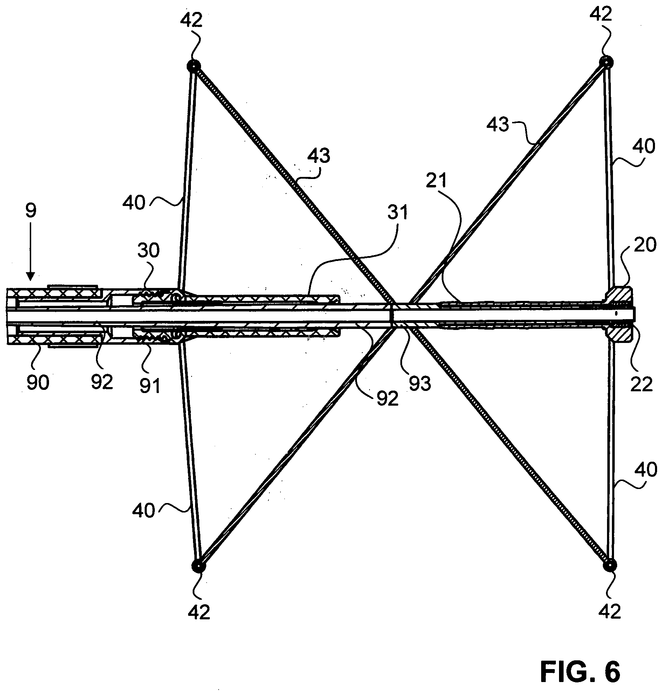

In Figur 3 ist dieser Okkluder nun im annähernd langgestreckten Zustand dargestellt. In diesem, bzw. in einem noch stärker langgezogenem und somit in quer zur Längsachse verlaufender Richtung kompakteren Zustand wird der Okkluder in den menschlichen oder tierischen Körper eingebracht. Hierfür wird ein Kathetersystem verwendet, wie dies im Stand der Technik bekannt ist. Ein Beispiel für eine derartige Einbringung ist in Figur 6 dargestellt.

Das Kathetersystem 9 ist in Figur 6 nur mit seinem distalen Ende wiedergegeben. Es weist einen hohlen Katheterkörper 90 auf, welcher vorzugsweise von einem flexiblen Schlauch gebildet ist. Auf diesem Katheterkörper 90 ist an seinem Ende ein steifer Kopf 91 mit einem Innengewinde aufgesteckt. Mit diesem Innengewinde wird der Katheter 9 mit dem Aussengewinde des proximalen Kopplungsteils 3 des Okkluders verbunden.

Im Katheterkörper 90 verläuft ein flexibler Katheterschlauch 92, welcher die Aufnahmehülse 31 des proximalen Kopplungsteils 3 durchsetzt und welche in eine Katheterspitze 93 übergeht. Diese Katheterspitze 93 reicht in den Einsteckschaft 21 des distalen Kopplungsteils 2 hinein. Sie ist mit einem Aussengewinde versehen, welches in ein Innengewinde 22 des distalen Kopplungsteils 2 eingreift (siehe Figur 5).

Der Okkluder wird komprimiert, indem eine über das Kathetersystem übertragene axiale Zugkraft auf die Verbindungsdrähte 43 aufgebracht wird. Durch Zurückziehen des distalen Endes des Schlauchs 92 zum Katheterkörper 90 hin, wird der Okkluder aufgespannt und expandiert. Die Expansion kann je nach Ausführungsform selbsttätig oder durch Krafteinwirkung erfolgen.

Ein derartiger expandierter Zustand ist in Figur 6 sichtbar. Der Okkluder lässt sich nun optimal im Durchgang platzieren. Zum Schluss wird der Schlauch 92 noch weiter zurückgezogen, so dass die zwei Kopplungsteile 2, 3 ineinander greifen und den Okkluder in der aufgespannten Position fixieren. Die Gewindeverbindungen zwischen Katheter und Okkluder werden gelöst und der Katheter wird entfernt.

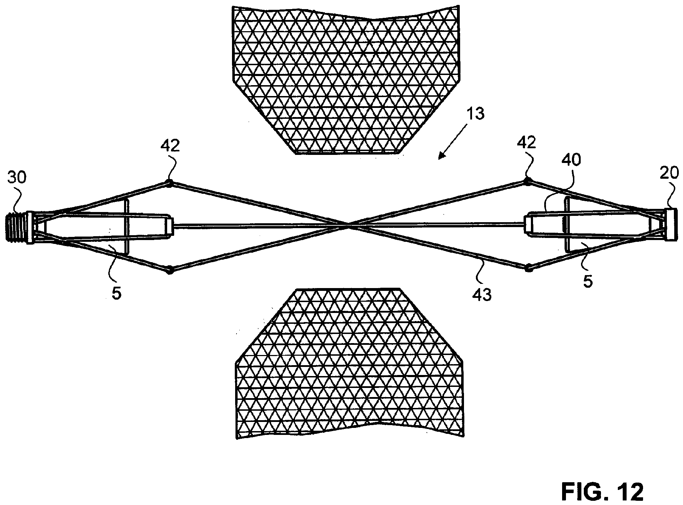

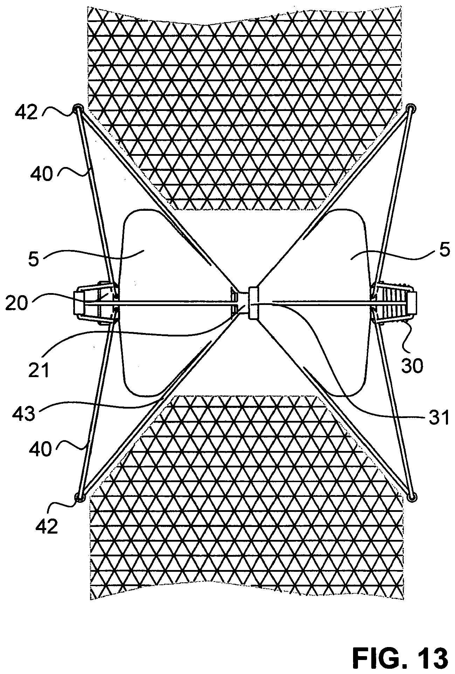

In den Figuren 12 und 13 ist der Okkluder im Defekt 13 dargestellt.

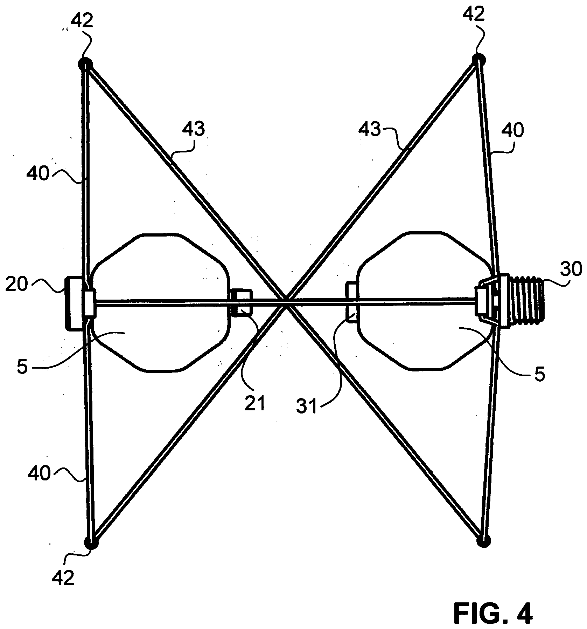

In den Figuren 3 und 4 ist ein erstes Beispiel eines Versschlusskörpers 5 dargestellt, wie er mit der oben beschriebenen Fixierungseinheit eingesetzt ist. Es handelt sich hier um einen komprimierbaren, flexiblen Tampon. In diesem Beispiel sind zwei Tampons 5 vorhanden, wobei ein erster Tampon 5 auf die Aufnahmehülse 3 des proximalen Kopplungsteils 3 und ein zweiter Tampon 5 auf den Einsteckschaft 21 des distalen Kopplungsteils 2 gesteckt ist. Vorzugsweise sind sie mit diesen Teilen 2, 3 verschweisst, vernäht oder verklebt. Sie können jedoch auch anderweitig fixiert sein. Als Tampon 5 eignet sich insbesondere ein schwammartiges Material oder ein gewebtes Material wie beispielsweise Gore-Tex®. Vorzugsweise wird ein Material verwendet, welches am Anfang blutdurchlässig ist, wobei die Thrombozyten im Material hängen bleiben und so der Verschlusskörper blutdicht wird und ein Einwachsen im Defekt iniziiert wird.

Wie in Figur 4 erkennbar ist, dehnen sich die Tampons 5 bei der Expansion der Fixierungseinheit 4 selbsttätig aus und sie passen sich der Defektform an.

Es kann auch nur auf einer Seite des Okkluders ein Tampon angeordnet sein.

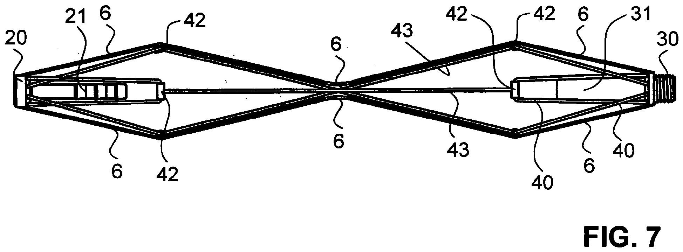

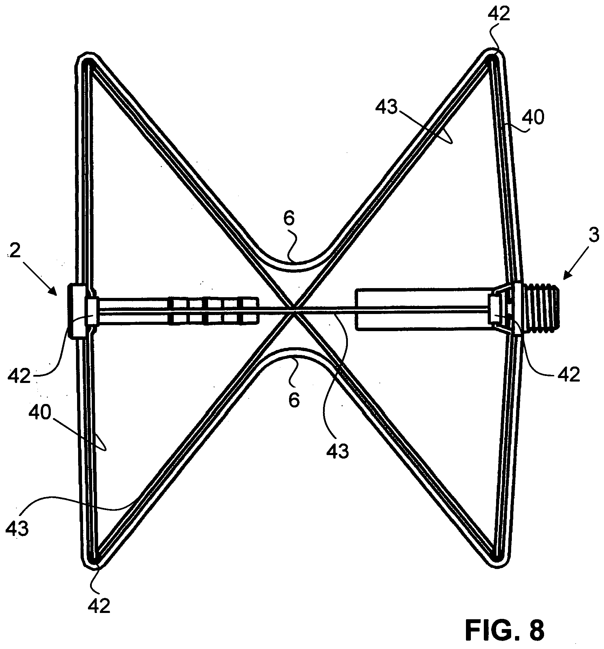

In den Figuren 7 und 8 ist ein anderer Verschlusskörper gezeigt. Es handelt sich hier um einen Ballon 6, welcher am distalen und proximalen Endstück 20, 30 befestigt ist und die Fixierungseinheit 4 umschliesst. Der Ballon 6 ist vorzugsweise als Doppelballon mit zwei Kammern und einem verjüngten Verbindungsbereich ausgebildet. Er wird vorzugsweise beim Aufstellen der Fixierungsarme 40 ausgedehnt, wie dies in Figur 8 erkennbar ist. Der Ballon besteht vorzugsweise aus PET, Polycarbonat, Polyvinylchlorid, PP oder Silikon. Die Ballonhülle kann auch innerhalb der Fixierungsarme 40 und somit der Fixierungseinheit 4 angeordnet sein.

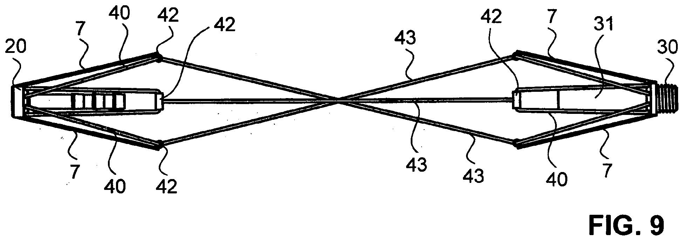

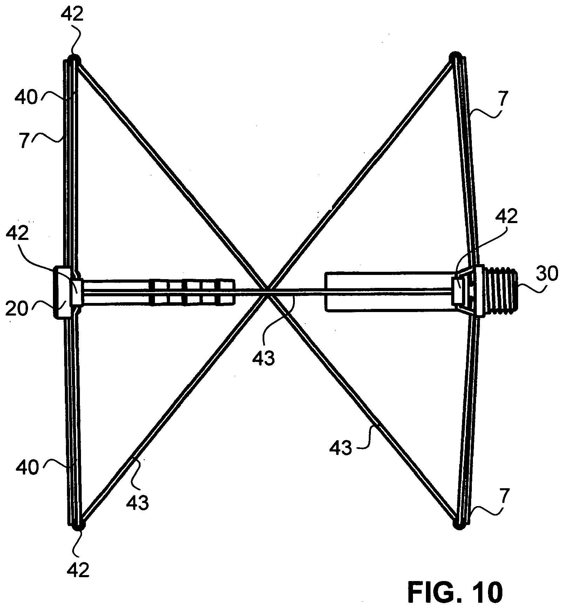

In den Figuren 9 und 10 ist eine weitere Variante eines Verschlusskörpers gezeigt. Es handelt sich hier um eine kreisförmige Membran 7, beispielsweise aus PET oder Dacron. Sie ist mit den Fixierungsarmen 40 der distalen oder proximalen Seite verbunden. In diesem Beispiel weisen sowohl die distalen wie auch die proximalen Fixierungsarme 40 je eine Membran 7 auf. Es kann jedoch auch nur auf einer Seite eine Membran 7 vorhanden sein. Vorzugsweise befindet sich die Membran 7 jeweils auf der dem entsprechenden Endstück 20, 30 zugewandten Seite der Fixierungsarme 40. Die Membran 7 kann an die Fixierungsarme 40 angenäht, angeklebt oder anderweitig mit ihren verbunden sein. Diese Membranen 7 werden, wie dies in Figur 10 erkennbar ist, mittels der Fixierungsarme 40 aufgespannt. Auch die Membranen können innerhalb der Fixierungseinheit 4, also auf der anderen Seite der Fixierungsarme 40 angeordnet sein.

Die genannten verschiedenen Verschlusskörper lassen sich auch in demselben Okkluder miteinander kombinieren.

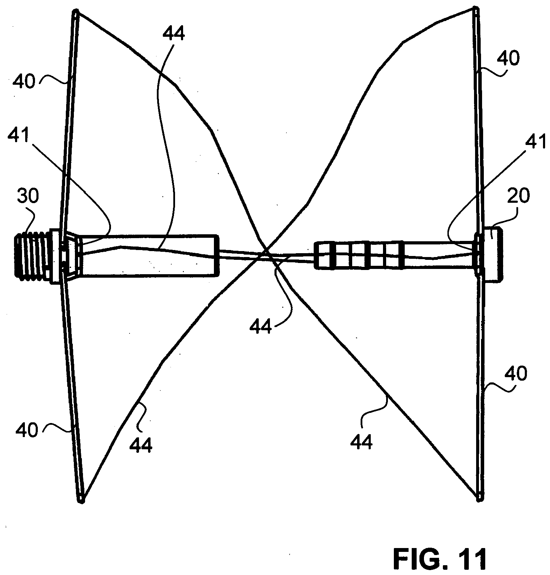

In Figur 11 ist ein weiteres Ausführungsbeispiel des erfindungsgemässen Okkluders dargestellt. Es ist im Wesentlichen gleich aufgebaut wie die oben beschriebenen Okkluder und es kann insbesondere die oben beschriebenen Verschlusskörper aufweisen. Der Okkluder weist jedoch keine Verbindungsdrähte 43 sondern Verbindungsschnüre 44 auf. Diese können wiederum an Hülsen 42 befestigt, insbesondere angeknüpft sein. Vorzugsweise sind jedoch keine Hülsen 42 vorhanden und die Fäden oder Schnüre 44 sind direkt an den Stegen der Fixierungsarme 40 befestigt, insbesondere angeknüpft oder verklebt. Damit sich in diesem Beispiel die Fixierungsarme 40 in die expandierte Position aufstellen, sind sie vorzugsweise in einem Winkel, wie oben beschrieben, in den Kopplungsteilen 2, 3 gehalten.

**BEZUGSZEICHENLISTE**

| | | | |
|---|---|---|---|
| 1 | Herz | 4 | expandierbare |
| 10 | rechter Vorhof | | Fixierungseinheit |
| 11 | untere Hohlvene | 40 | Fixierungsarm |
| 12 | rechte Kammer | 401 | Nagelkopf |
| 13 | Defekt in der Kammerscheidewand (VSD) | 41 | Steg |
| | | 42 | Hülse |
| 14 | linke Kammer | 43 | Verbindungsdraht |
| 15 | Weg des Führungsdrahtes | 44 | Verbindungsschnur |
| 16 | linker Vorhof | | |
| 17 | obere Hohlvene | 5 | Tampon |
| 2 | distales Kopplungsteil | 6 | Ballonhülle |
| 20 | distales Endstück | | |
| 21 | Einsteckschaft | 7 | Membran |
| 22 | Innengewinde | | |
| 23 | Sackloch | 9 | Kathetersystem |
| 24 | Aufnahmeöffnung | 90 | Katheterkörper |
| | | 91 | Kopf |
| 3 | proximales Kopplungsteil | 92 | Katheterschlauch |
| 30 | proximales Endstück | 93 | Katheterspitze |
| 31 | Aufnahmehülse | | |

## Patentansprüche

1. Okkluder zum Verschliessen eines Durchgangs in einem Kreislaufsystem, wobei der Okkluder eine expandierbare Fixierungseinheit (4) zur Fixierung des Okkluders beim Durchgang aufweist, wobei der Okkluder im Durchgang von einer kompakten Erscheinungsform in eine expandierte Erscheinungsform überführbar ist und wobei der Okkluder ein distales und ein proximales Axialteil (2, 3) aufweist, in welchen die Fixierungseinheit (4) schwenkbar gehalten ist, wobei die Fixierungseinheit (4) distale Fixierungsarme (40) und proximale Fixierungsarme (40) aufweist, wobei die distalen Fixierungsarme (40) im distalen Axialteil (2) und die proximalen Fixierungsarme (40) im proximalen Axialteil (3) schwenkbar gehalten sind, und wobei die distalen und die proximalen Fixierungsarme (40) freie Enden aufweisen, welche über relativ zu den Fixierungsarmen (40) bewegbare Verbindungsglieder (43, 44) miteinander verbunden sind, wobei je ein distaler Fixierungsarm (40) mit je einem proximalen Fixierungsarm (40) verbunden ist, **dadurch gekennzeichnet, dass** jeder dieser Fixierungsarme (40) u-förmig ausgebildet ist mit zwei Schenkeln und einem diese Schenkel verbindenden Steg (41), wobei die zwei Schenkel freie Enden aufweisen, welche im proximalen oder distalen Axialteil (2, 3) schwenkbar gehalten sind und wobei der Steg (41) das besagte freie Ende des Fixierungsarms (40) bildet.

2. Okkluder nach Anspruch 1, wobei die Verbindungsglieder (43, 44) zwischen den einzelnen distalen und proximalen Fixierungsarmen (40) unabhängig voneinander verlaufen.

3. Okkluder nach einem der Ansprüche 1 oder 2, wobei sich die Verbindungsglieder (43, 44) in einem Bereich kreuzen und wobei sie in diesem Bereich einzeln verschiebbar in einer sie gemeinsam umgebenden Hülse (42) verlaufen.

4. Okkluder nach einem der Ansprüche 1 bis 3, wobei der Steg (41) annähernd geradlinig ausgeführt ist.

5. Okkluder nach einem der Ansprüche 1 bis 4, wobei der Steg (41) von einer Hülse (42) umfasst ist und wobei der Steg (41) schwenkbar in der Hülse (42) gehalten ist.

6. Okkluder nach einem der Ansprüche 1 bis 5, wobei die Fixierungsarme (40) Drähte sind.

7. Okkluder nach einem der Ansprüche 1 bis 6, wobei die Verbindungsglieder (43, 44) Drähte oder Schnüre sind.

8. Okkluder nach einem der Ansprüche 1 bis 7, wobei er mindestens einen Verschlusskörper (5, 6, 7) zum Verschliessen des Durchgangs aufweist und wobei der Verschlusskörper vorzugsweise eine Membran (7), ein Tampon (5) oder ein Ballon (6) ist.

9. Okkluder nach Anspruch 8, wobei als Verschlusskörper eine Membran (7) vorhanden ist und wobei diese Membran (7) mittels der Fixierungsarme (40) in die expandierte Erscheinungsform bringbar ist.

10. Okkluder nach Anspruch 8, wobei als Verschlusskörper ein komprimierbarer elastischer Tampon (5) vorhanden ist.

11. Okkluder nach Anspruch 8, wobei als Verschlusskörper ein Ballon (6) vorhanden ist, welcher sich vom proximalen Axialteil (3) bis zum distalen Axialteil (2) erstreckt, an diesen zwei Teilen (2, 3) befestigt ist und die Fixierungsarme (40) und Verbindungsglieder (43, 44) umgibt.

12. Okkluder nach Anspruch 8, wobei der Verschlusskörper (5, 6, 7), vorzugsweise ein Tampon (5), in einem Bereich angeordnet ist, welcher von einer Seite von den distalen Fixierungsarmen (40) und an einer gegenüberliegenden Seite von den proximalen Fixierungsarmen (40) definiert ist.

## Claims

1. An occluder for closing a passage in a circulatory system, wherein the occluder has an expandable fixation unit (4) for fixing the occluder on the passage, wherein the occluder can be transferred in the passage from a compact form of appearance to an expanded form of appearance, and wherein the occluder has a distal and a proximal axial part (2, 3), in which parts the fixation unit (4) is held pivotably, wherein the fixation unit (4) has distal fixation arms (40) and proximal fixation arms (40), wherein the distal fixation arms (40) are held pivotably in the distal axial part (2) and the proximal fixation arms (40) are held pivotably in the proximal axial part (3), and wherein the distal and the proximal fixation arms (40) have free ends, which are connected to one another via connecting members (43, 44) that are movable relative to the fixation arms (40), wherein in each case a distal fixation arm (40) is connected to a proximal fixation arm (40), **characterized in that** each of these fixation arms (40) is U-shaped with two branches and a bridge (41) joining these branches, wherein the two branches have free ends, which are held pivotably in the proximal or distal axial part (2, 3), and wherein the bridge (41) forms said free end of the fixation arm (40).

2. The occluder as claimed in claim 1, wherein the connecting members (43, 44) extend independently of one another between the individual distal and proximal fixation arms (40).

3. The occluder as claimed in either of claims 1 and 2, wherein the connecting members (43, 44) intersect in one area, and wherein, in this area, they extend in an individually displaceable manner in a sleeve (42) that surrounds them jointly.

4. The occluder as claimed in one of claims 1 through 3, wherein the bridge (41) is approximately rectilinear.

5. The occluder as claimed in one of claims 1 through 4, wherein the bridge (41) is enclosed by a sleeve (42), and wherein the bridge (41) is held pivotably in the sleeve (42).

6. The occluder as claimed in one of claims 1 through 5, wherein the fixation arms (40) are wires.

7. The occluder as claimed in one of claims 1 through 6, wherein the connecting members (43, 44) are wires or strings.

8. The occluder as claimed in one of claims 1 through 7, wherein it has at least one closure body (5, 6, 7) for closing the passage, and wherein the closure body is preferably a membrane (7), a tampon (5) or a balloon (6).

9. The occluder as claimed in claim 8, wherein a membrane (7) is present as closure body, and wherein this membrane (7) can be brought to the expanded form of appearance by means of the fixation arms (40).

10. The occluder as claimed in claim 8, wherein a compressible elastic tampon (5) is present as closure body.

11. The occluder as claimed in claim 8, wherein a balloon (6) is present as closure body, which balloon extends from the proximal axial part (3) to the distal axial part (2), is secured to these two parts (2, 3) and surrounds the fixation arms (40) and connecting members (43, 44).

12. The occluder as claimed in claim 8, wherein the closure body (5, 6, 7), preferably a tampon (5), is arranged in an area that is defined on one side by the distal fixation arms (40) and on an opposite side by the proximal fixation arms (40).

## Revendications

1. Dispositif d'occlusion pour fermer un passage dans un système circulatoire, le dispositif d'occlusion présentant une unité de fixation expansible (4) pour fixer le dispositif d'occlusion au niveau du passage, le dispositif d'occlusion dans le passage pouvant être amené d'une forme ayant un aspect compact dans une forme ayant un aspect expansé et le dispositif d'occlusion présentant une partie axiale distale (2) et une partie axiale proximale (3), dans lesquelles l'unité de fixation (4) est retenue de manière pivotante, l'unité de fixation (4) présentant des bras de fixation distaux (40) et des bras de fixation proximaux (40), les bras de fixations distaux (40) étant retenus de manière pivotante dans la partie axiale distale (2) et les bras de fixation proximaux (40) étant retenus de manière pivotante dans la partie axiale proximale (3), et les bras de fixation distaux et proximaux (40) présentant des extrémités libres qui sont connectées les unes aux autres par le biais d'organes de liaison (43, 44) pouvant être déplacés par rapport aux bras de fixation (40), un bras de fixation distal respectif (40) étant connecté à un bras de fixation proximal respectif (40), **caractérisé en ce que** chacun de ces bras de fixation (40) est réalisé en forme de U avec deux branches et une âme (41) reliant ces branches, les deux branches présentant des extrémités libres qui sont retenues de manière pivotante dans la partie axiale proximale ou distale (2, 3) et l'âme (41) formant ladite extrémité libre du bras de fixation (40).

2. Dispositif d'occlusion selon la revendication 1, dans lequel les organes de liaison (43, 44) s'étendent indépendamment les uns des autres entre les bras de fixation individuels distaux et proximaux (40).

3. Dispositif d'occlusion selon l'une quelconque des revendications 1 ou 2, dans lequel les organes de liaison (43, 44) se croisent dans une région et s'étendent dans cette région de manière déplaçable individuellement dans une douille (42) qui les entoure en commun.

4. Dispositif d'occlusion selon l'une quelconque des revendications 1 à 3, dans lequel l'âme (41) est réalisée sous forme approximativement rectiligne.

5. Dispositif d'occlusion selon l'une quelconque des revendications 1 à 4, dans lequel l'âme (41) est entourée par une douille (42) et l'âme (41) est retenue de manière pivotante dans la douille (42).

6. Dispositif d'occlusion selon l'une quelconque des revendications 1 à 5, dans lequel les bras de fixation (40) sont des fils métalliques.

7. Dispositif d'occlusion selon l'une quelconque des revendications 1 à 6, dans lequel les organes de liaison (43, 44) sont des fils métalliques ou des cordons.

8. Dispositif d'occlusion selon l'une quelconque des revendications 1 à 7, présentant au moins un corps de fermeture (5, 6, 7) pour fermer le passage et dans lequel le corps de fermeture est de préférence une membrane (7), un tampon (5) ou un ballonnet (6).

9. Dispositif d'occlusion selon la revendication 8, dans lequel le corps de fermeture est une membrane (7) et cette membrane (7) peut être amenée à la forme d'aspect expansé au moyen des bras de fixation (40).

10. Dispositif d'occlusion selon la revendication 8, dans lequel le corps de fermeture est un tampon élastique compressible (5).

11. Dispositif d'occlusion selon la revendication 8, dans lequel le corps de fermeture est un ballonnet (6) qui s'étend depuis la partie axiale proximale (3) jusqu'à la partie axiale distale (2), qui est fixée à ces deux parties (2, 3) et qui entoure les bras de fixation (40) et les organes de liaison (43, 44).

12. Dispositif d'occlusion selon la revendication 8, dans lequel le corps de fermeture (5, 6, 7), de préférence un tampon (5), est disposé dans une région qui est définie d'un côté par les bras de fixations distaux (40) et, du côté opposé, par les bras de fixation proximaux (40).
